# EUROPÄISCHE PATENTSCHRIFT

(11) **EP 2 260 784 B1**
(45) Veröffentlichungstag und Bekanntmachung des Hinweises auf die Patenterteilung: **03.08.2016**
(21) Anmeldenummer: 10005943.5
(22) Anmeldetag: 09.06.2010
(51) Int. Cl.: A61B 90/00

(54) **System zur Orientierungsunterstützung und Darstellung eines Instruments im Inneren eines Untersuchungsobjektes insbesondere im menschlichen Körper**
System for orientation support and imaging an instrument in the interior of an examined object, particularly in the human body
Système d'aide à l'orientation et représentation d'un instrument à l'intérieur d'un objet d'examen, notamment dans le corps humain

(30) Priorität: 10.06.2009 DE 102009025077
(43) Veröffentlichungstag der Anmeldung: 15.12.2010
(73) Patentinhaber: Karl Storz GmbH & Co. KG, 78532 Tuttlingen (DE)
(72) Erfinder: Marescaux, Jacques, 67310 Scharrachbergheim (FR); Irion, Klaus M., 78532 Tuttlingen (DE)

(56) Entgegenhaltungen:
- EP-A1- 1 738 709
- EP-A1- 1 982 650
- JP-A- 2000 350 734
- US-A1- 2006 281 971
- US-A1- 2007 002 038

## Beschreibung

Die vorliegende Erfindung bezieht sich auf ein System zur Orientierungsunterstützung und Darstellung eines Instruments im Inneren eines Untersuchungsobjektes insbesondere eines Endoskops im menschlichen oder tierischen Körper.

Es sind bereits verschiedene Verfahren und Vorrichtungen bekannt, die die Kombination minimal-invasiver endoskopischer Operationsmethoden mit Techniken der Augmented-Reality und der bilddatengestützten Navigation zeigen (R. Mösges, Die Methodik computergestützten Operierens. Habilitationsschrift, 1992, Hochschule Aachen).

Dabei werden zunächst basierend auf präoperativ akquirierten Bilddaten eines 3D-CT- oder MRT-Scanners die dreidimensionale Darstellung der Organsysteme (individuelle Patientenanatomie im Körperinneren) ermittelt und auf einem Monitor dargestellt. Die Position und die Orientierung zum Beispiel eines Endoskops wird dann während der Intervention in das dreidimensionale Bild des Körperinneren ein- bzw. abgebildet.

In der WO 2006/116597 A2 wird ein System zur Navigationsunterstützung von medizinischen Instrumenten beschrieben. Hier wird ein Endoskop, welches mit einem Lagesensor versehen ist im Körper eines Menschen anhand seiner 3D-Positionen verfolgt. Dabei werden zunächst mittels eines Computertomographen Bilder aufgenommen, um daraus ein virtuelles Bild vom Inneren des Körpers zu erstellen. Das virtuell erstellte Bild wird dann auf einem Bildschirm dargestellt. Ebenso wird ein Symbol des Endoskops auf diesem Bildschirm mit eingeblendet. Nachteilig jedoch ist, dass die gesamte Länge des Instruments nicht dargestellt wird und schlecht erfassbar ist.

In Dokument D1 (US 2007/0002038 A1) ist ein Verfahren und eine Vorrichtung zur Verfolgung und verlaufsentsprechenden Darstellung einer in den Körper eines Menschen aufgenommenen Kapsel, die im Inneren mit einem Magneten versehen ist, beschrieben. Mittels eines Positionierungssystems und Sensoren, die sich auf dem Körper des Patienten angebracht sind, werden durch Triangulation die 3D Lagedaten des Instruments ermittelt, um schließlich eine symbolisierte Darstellungen der aufgenommenen Kapsel zu generieren. Weiterhin wird mit den auf dem Körper des Patienten angebrachten Sensoren ein Körperoberflächenbild generiert. Diese generierten Darstellungen werden dann gemeinsam auf einem Monitor dargestellt. Der Arzt erhält somit während einer Untersuchung Information darüber, wo sich die Kapsel im Körper des Patienten befindet. Er erhält jedoch keine Bildinformationen des Instruments, die, bezogen auf die Bilddaten des Körpers, größengerecht sind.

In Dokument D2 (EP 1738709 A1) ist ein Gerät zur Operationsunterstützung beschrieben. Während des Eingriffs wird mittels einer Videokamera Aufnahmen des Operationsfeldes genommen. Die Kamera ist jedoch nicht dazu ausgelegt, die Bildinformationen auf einem Bildschirm dazustellen.

Dokument US2006/281971, das als nächstliegender Stand der Technik angesehen wird, offenbart alle Merkmale gemäß dem Oberbegriff des unabhängigen Anspruchs 1.

Der Nachteil all dieser Verfahren ist, dass um eine Augmented-Reality-Anwendung aufbauen zu können, zuerst präoperativ ein teures und aufwändig zu bedienendes Gesamtsystem, bestehend aus Kamera, Trackingeräte, Kernspintomographen, Unterstützungssoftware usw. bereitgestellt werden muss. Zudem haben die ebengenannten Methoden den großen Nachteil, dass vor und ggf. auch während einer einzigen Operation, zur Erstellung des virtuellen Bildes des Körperinneren mittels eines Computertomographen (CT), eine Vielzahl von Röntgenbildern benötigt wird. Das bedeutet eine hohe Strahlenbelastung für den Patienten und auch für das OP-Personal. Des Weiteren ist im Fall einer Notoperation oft nicht mehr die Zeit gegeben, entsprechende 3D-Bilddaten aus einer Computertomographie präoperativ bereitstellen zu können. Was zur Folge hat, dass dem Arzt bei diesen Prozeduren wichtige Informationen für die räumliche Orientierung fehlen.

Es ist daher Aufgabe der vorliegenden Erfindung, ein System zur Orientierung in Körpern bereitzustellen, das die Strahlenbelastung vor und während eines Eingriffs reduziert, das die Echtzeitanforderung möglichst erfüllt, das leicht bedienbar ist und insbesondere einem unerfahrenen Arzt eine verbesserte und schnelle Orientierung und Darstellung eines in einen Körper eingeführten Instruments ermöglicht.

Diese Aufgabe wird gemäß der Erfindung durch die Merkmale der unabhängigen Ansprüche gelöst. Die abhängigen Ansprüche bilden weitere Gedanken der Erfindung in besonders vorteilhafter Weise weiter.

Erfindungsgemäß ist also ein System zur Orientierungsunterstützung und Darstellung von medizinischen Instrumenten in Untersuchungsobjekten, wie beispielsweise einen menschlichen Körper, mit den genannten Merkmalen vorgesehen.

Das System weist eine Datenerfassungseinheit insbesondere eine Kameravorrichtung auf, die vorzugsweise eine Bildverarbeitung beinhaltet, und die zumindest ein Teil der Körperoberfläche erfasst und Bilddaten der Körperoberfläche generiert, wobei währenddessen mittels eines Lagemeßsystems 3D-Lagedaten insbesondere die Position und ggfs. Orientierung (im Raum) eines in das Innere eines Untersuchungsobjektes insbesondere eines Körpers eingeführten Instrumentes insbesondere eines Endoskops erfasst wird. Mittels der 3D-Lagedaten wird ein virtuelles Modell des in das Untersuchungsobjekt eingeführten Instruments gewonnen, wobei insbesondere eine Mehrzahl an 3D-Lagedaten von unterschiedlichen Abschnitten des Untersuchungsobjektes insbesondere des Endoskops erfasst werden.

Das System zeigt eine Auswerteeinheit, welche die zugeführten 3-D Lagedaten mit den zugeführten Bilddaten so verknüpft, dass ein den Bilddaten lage- und größenentsprechendes virtuelles Bild des Instruments generiert wird und dieses vorzugsweise mit den Bilddaten der Datenerfassungseinheit bzw. Kameravorrichtung verknüpft werden, sodass vorzugsweise ein gemeinsames Augmented-Reality-Bild geschaffen wird.

Schließlich werden die aus dem Körperoberflächenbild und den 3D-Lagedaten gewonnenen Bildinformationen einer Ausgabeeinheit zugeführt und mittels dieser, zum Beispiel auf einem Monitor, dargestellt. Das erstellte Bild des Instrumentes bzw. Endoskops wird dabei lage- und größenentsprechend vorzugsweise in das Körperoberflächenbild in der Art eines Augmented-Reality-Bildes integriert dargestellt.

Das dargestellte Bild, zeigt einerseits die Oberfläche des Untersuchungsobjektes bzw. Körpers des Patienten und andererseits ein an die Größe und Lage des Untersuchungsobjektes bzw. Körpers des Patienten angepasstes virtuelles Bild des Instruments bzw. Endoskops. Mit dem erfindungsgemäßen System wird bevorzugt zumindest ein Körperbild in Echtzeit mit dem erstellten virtuellen Endoskopbild verknüpft und dargestellt.

Durch dieses System gelingt es einerseits eine sehr intuitive und schnelle Erfassung der Orientierung des Instrumentes auch unter schwierigen äußeren Umständen, z.B. im Verlauf einer langen und schwierigen Operation oder endoskopischen Untersuchung unter Zeitdruck zu gewinnen. Dies ermöglicht eine deutlich risikoärmere Untersuchung bzw. Operation eines Patienten, da Behandlungsfehler aufgrund falscher Orientierung weitgehend ausgeschlossen werden. Die Orientierung wird gerade durch die Verknüpfung der virtuellen Informationen zur Lage des Instrumentes mit der wohl bekannten Oberfläche des Untersuchungsobjektes besonders geprägt. Belastende und zeitaufwendige präoperative CT-Untersuchungen sind damit nicht mehr zwingend.

Erfindungsgemäß kann auch ein typisches Problem vermieden werden, das darauf beruht, dass die präoperativ gewonnenen Planungsdaten nicht mehr mit dem aktuellen Situs übereinstimmen. So kann aufgrund einer zwischenzeitlich Organverformung, die nach der Erhebung der Informationen zur Durchführung eines computergestützten Eingrifft eingetretenen ist, ein gänzlich neues Operationsumfeld gegeben sein. Im Bereich der Weichteiloperationen kann es auch durchaus vorkommen, dass durch eine intraoperative Bewegung der weichen Organe die Umsetzung der Operationsplanung durch ein Navigationssystem nicht mehr möglich ist. Diese Probleme werden durch das erfindungsgemäße System zumindest anteilig überwunden, da die gesteigerte Orientierung teilweise ausreichend ist, erfolgreich die Operation abzuschließen. Zudem kann dies erfindungsgemäß in Echtzeit erfolgen, was bei den bekannten Systemen mit präoperativ erzeugten Datensätzen nicht möglich ist.

In einer weiteren bevorzugten Ausgestaltung des Systems wird das zumindest eine virtuelle Bild des Instruments insbesondere eines Endoskops auf eine Projektionsfläche im Untersuchungsraum projiziert. Dabei kann die Projektionsfläche insbesondere eine Wand eines Operationssaales also innerhalb des sterilen Bereiches darstellen, während die Projektionseinheit außerhalb des sterilen Bereiches durch eine Glasscheibe von diesem getrennt angeordnet ist. Dies ermöglicht eine effiziente Sterilisierung des Systems ohne die verbesserte Orientierungsfähigkeit zu beieinträchtigen.

Alternativ und bevorzugt wird auf die typisch nicht ebene Oberfläche des Untersuchungsobjekts insbesondere eines Körpers projiziert. Das System weist dabei zusätzlich eine Einheit zur Erfassung der Topographie des wenigstens durch die Kameravorrichtung erfassten Bereiches des Untersuchungsobjekts auf. Die Topographiedaten werden neben den Bilddaten und 3D-Lagedaten einer Auswerteeinheit zuführt. Im Rahmen der Auswertung werden dann Bilddaten generiert, die großen-, lage- und projektionsflächengerecht ein reelles Abbild des Instrumentes als virtuelles Bild generiert. Damit ist gewährleistet, dass das projizierte Bild auf der Oberfläche des Untersuchungsobjektes realitätsnah erscheint und dadurch eine sehr intuitive fehlerarme und belastungsarme Orientierung des Benutzers insbesondere des Operateurs ermöglicht. Insbesondere gelingt es eine anatomisch und lagekorrekte Darstellung des Instruments auf dem Untersuchungsobjekt zu ermöglicht, was gerade im Hinblick auf das Einbringen eines weiteren Instrumentes durch die Oberfläche des Untersuchungsobjektes an einer durch die Projektion definierten Stelle verlässlich ermöglicht.

Eine Projektion des virtuell erstellten Bildes des in den Körper eingeführten Endoskops auf die Oberfläche des Untersuchungsobjekts insbesondere auf die Köperoberfläche eines Patienten hat den Vorteil, dass ein Arzt, nachdem er das Instrument angesetzt und in eine Körperöffnung des Patienten eingeführt hat, die aktuelle Lage des Instruments insbesondere online auf dem Patienten verfolgen kann und er nicht während des Eingriffs Orientierungs- oder Lageinformation auf einem abgesetzten, zusätzlichen Bildschirm erhält sondern direkt beim Blick auf den Patienten. Für einen Arzt entsteht somit der Eindruck, als blicke er quasi durch die Haut hindurch direkt auf das eingeführte Instrument. Er wird somit auch nicht unnötig abgelenkt und muss also nicht ständig den Kopf weit vom Patienten zum Bildschirm weg- und wieder zurückdrehen, was sich sehr positiv auf die Orientierungsfähigkeit auswirkt

Vorzugsweise wird die Erfassung der Topographie mittels eines Streifenlichtprojektionsverfahren bestimmt.

In einer weiteren bevorzugten Ausgestaltung des erfindungsgemäßen Systems weist das Lagemeßsystem zur Erfassung der Position und Orientierung des Instruments wenigstens eine insbesondere mehr als 3 Sensoreinheiten auf. Diese sind bevorzugt als elektromagnetische Spulen (Sende- oder Empfangsspulen) ausgebildet, mittels der die Lage und Orientierung in einem äußeren Elektro-magnetischen Feld ermittelt wird. Ein Beispiel für eine derartige Sensoreinheit ist durch das Produkt "Aurora System" des Unternehmens NDI, das im flyer "NDI Aurora Electromagnetic Tracking System" gezeigt ist, realisiert. Alternativ haben sich Magnetfeldsensoren und Trägheitsmesssysteme bewährt. Werden eine Reihe von insbesondere elektromagnetischen Spulen auf der Oberfläche des Instrumentes oder in dieses integriert verteilt und über dessen Länge angeordnet, so läst sich sehr verlässlich der Verlauf des eingeführten Instrumentes bestimmen und darstellen. Gerade die elektromagnetischen Spulen ermöglichen dabei auf einfache Weise eine verlässliche und gleichzeitige Bestimmung der jeweiligen Lage im Raum.

Die Sensoreinheiten des erfindungsgemäßen Systems sind vorzugsweise gleichmäßig über einen Abschnitt des Instruments verteilt, was eine sehr gute Orientierung über die Gesamtlage des Instrumentes ermöglicht. Da jede Sensoreinheit fest mit dem Instrument insbesondere dem Schaft des Endoskops korreliert ist, lässt sich aus der 3D-Lage einer Sensoreinheit verlässlich auf die Position des Instrumentes schließen.

Vorzugsweise werden die Sensoreinheiten mit unterschiedlicher Dichte mit dem Instrument verbunden angeordnet. Insbesondere ist die Dichte im distalen Endbereich eines Endoskops erhöht, was zu einer noch verlässlicheren Information über die Lage des Instrumentes im Arbeitsbereich führt. Gerade bei flexiblen Endoskopen ist in diesem Bereich der Vorteil einer sicheren Orientierung gerade für weniger routinierte Operateure sehr wichtig, da dieser in seiner Orientierung besonders stark variieren kann und dadurch Fehlorientierungen und daraus folgende Fehlbehandlungen vermieden werden können.

Bei dem erfindungsgemäßen System können die Sensoren auch integriert in oder auf endoskopischen Instrumenten für die endoskopische Chirurgie angebracht sein. Dabei können solche Instrumente beispielsweise Zangen oder HF-Schneidgeräte sein. In einer weiteren Ausführungsform der Erfindung kann eine Sensoreinheit in eine Endoskopiekapsel (Endopille) integriert sein. Diese wird Kapsel wird vom Patienten eingenommen und durchwandert den Körper des Patienten. Dabei wird das Innere des Körpers erfasst und dokumentiert. Durch das erfindungsgemäße System wird die Endoskopiekapsel in ihrer Lage im Körper zur besseren Orientierung des Operateurs in Verbindung mit dem Körper dargestellt. Sollte eine operative Entnahme der Endoskopiekapsel nötig sein, so ist anhand der erfindungsgemäßen Darstellung der Endoskopiekapsel der geeignete Ort für den Eingriff verlässlich bestimmt werden. Gerade bei einer erfindungsgemäßen Projektion auf den Körper zeigt sich dieser Vorteil sehr plastisch.

Auch hat es ich bewährt, das System mit einen Instrument auszubilden, das ein Implantat insbesondere ein Kurzzeitimplantat darstellt. Mit Hilfe des erfindungsgemäßen Systems lässt sich das Einbringen des Instrumentes in den Körper sowie den verbleib an der gewünschten Position sowie das Entfernen oder entnehmen des als Implantat ausgebildeten Instrumentes verlässlich verfolgen.

Des weitern können zusätzliche Sensoreinheiten extrakorporal angeordnet sein, beispielsweise am Untersuchungsobjekt selbst oder im Untersuchungsraum vorzugsweise an einer Wand desselben, was eine genauere Referenzierung und damit Lageerkennung des eingeführten Instruments ermöglicht. Besonders bevorzugt haben sich extrakoporale Sensoren, die am Eintrittsbereich des Instruments in das Untersuchungsobjekte angeordnet sind und damit auch durch die Datenerfassungseinheit parallel erfasst werden. Eine besonders sichere Referenzierung und damit sichere Lage- und orientierungsrichtige Darstellung wird dadurch ermöglicht.

Besonders vorteilhaft werden Referenzmarker verwendet, die nicht nur eine Referenzierung für eine Komponente (z.B. Datenerfassungseinheit, Lagemeßsystem oder Einheit zur Erfassung der Topographie) des erfindungsgemäßen Systems ermöglicht sondern zugleich in mehreren eine Referenzierung und vorzugsweise eine gemeinsame einheitliche Referenzierung ermöglicht. Dies führt zu einer verbesserten Weiterverarbeitung der erfassten Daten, die auf einer gemeinsamen Referenz (einheitliche Raumkoordinaten) aufsetzen.

Gerade bei dünnen Instrumenten wie bei Kathetern für Operationen zeigt das erfindungsgemäße System seine Stärken, da diese teilweise nur schwierig zielgerichtet geführt werden können und bei einer Fehlführung relevante Schäden bei den Patienten erzeugen können. Eine sichere Orientierung ist hier besonders wichtig. Dabei sind die Sensoreinheiten dergestalt angeordnet, dass beim Vorschieben des Instruments im Untersuchungsobjekt alle Bewegungen und jeweilige Abwinkelungen, das heißt alle Positions- und alle Lageänderungen, durch das Lagemeßsystem erfasst und daraus die aktuelle Position und Orientierung des Instruments ermittelt werden können.

Weiterhin wird die Erfassung der Bilddaten durch die Datenerfassungseinheit und die Erfassung der 3D-Lagedaten durch das Lagemeßsystem bevorzugt gleichzeitig und damit synchron durchgeführt. Die anschließende Auswertung dieser Daten sichert eine aussagekräftige Darstellung, welche keine unerwünschten Verzerrungen durch Zeitdiskontinuitäten der Daten zeigt. Die Auswertung beziehungsweise die Darstellung können dabei zu einem späteren Zeitpunkt insbesondere diskontinuierlich insbesondere nicht in Echtzeit erfolgen.

Wird dagegen wie bevorzugt die Erfassung und Auswertung wie die Darstellung kontinuierlich und synchron durchgeführt, so wird immer eine exakte Lagebestimmung und Erstellung eines virtuellen Modells des Endoskops in Verbindung mit den Bilddaten unabhängig von der jeweiligen Veränderung der Lage des Untersuchungsobjektes und des Instrumentes im Untersuchungsobjekt insbesondere durch das Vorschieben erreicht. Vorzugsweise werden die Zeitabstände kürzer als eine Sekunde sein und vorzugsweise im Bereich von Millisekunden oder darunter liegen. Die zeitliche Steuerung des Lagemeßsystems und der Datenerfassungseinheit und ggfs. der Einheit zur Erfassung der Topographie wird automatisiert insbesondere in Abhängigkeit der Untersuchungssituation gewählt und nicht durch den Operateur vorgenommen.

Vorzugsweise werden einzelne Positionen des Instruments beziehungsweise des Bereichs für das Einführen des Instruments in das Untersuchungsobjektes (Eintrittskanal), die sowohl vom Meßsystem als auch vom Datenerfassungssystem erfasst werden, als extrakoporale Bezugspunkt bzw. Bezugspunkte für die lage- und größenrichtige Generierung der Bilddaten verwendet. Hierdurch lässt sich eine einfache und schnelle Bilddatenverarbeitung gewährleisten, da diese Bezugspunkte Fixpunkte in der Anpassung der Bilddaten darstellen und dadurch der Rechenaufwand erheblich reduziert werden kann. Gerade hierdurch lassen sich Echtzeitsysteme auch ohne aufwändige Rechnersysteme realisieren.

Durch die Erfindung wird ein System zur Orientierungsunterstützung von medizinischen Instrumenten geschaffen und bereitgestellt, das es einem ungeübten/unerfahrenen Nutzer ermöglicht, von einem in den Körper eingeführten Instruments, zum Beispiel Endoskop, mittels virtueller Bildgebung (Augmented Reality) und passend zur aktuellen Endoskopbewegung und Endoskoppositionierung vorzugsweise in Echtzeit eine realitätsnahe bzw. lagerichtige Lokalisierung des navigierten Instruments in Verbindung mit dem Abbild des Äußeren des Patienten zu erhalten.

Der Nutzer erhält somit während des Vorschiebens des Instruments im Körper passend genau entweder direkt auf den Körper des Menschen projiziert oder in ein extrakorporal aufgenommenes Körperbild des Patienten eingeblendet auf einem Monitor eine visuelle Information über die aktuelle Lage/Position des Instruments im Körper des Menschen. Er erhält somit ein sicheres, schnelles System, das sich bedeutend weniger strahlungsbelastend auf den Patienten auswirkt.

Relevant sind diese Art der Systeme zur Orientierungsunterstützung und Darstellung eines Instruments bei neuen endoskopischen Techniken wie der NOTES-Chirurgie oder beim Einsatz der endoskopischen Kapsel oder Pille (Endopille).

Das erfindungsgemäße System zur Orientierungsunterstützung und Darstellung eines Instruments im Inneren eines Untersuchungsobjektes insbesondere eines Endoskops sowie die Funktionsweise der einzelnen Einheiten werden nachfolgend anhand einzelner Ausführungsbeispiele in Verbindung mit den Zeichnungen näher erläutert. Die Erfindung ist nicht auf diese Ausführungen beschränkt.

Hierbei zeigen:
Fig. 1 eine schematische Zeichnung eines erfindungsgemäßen Systems,
Fig.2 ein Endoskop mit einer erfindungsgemäßen Anordnung mehrerer Sensoreinheiten und
Fig. 3 ein Blockschaltbild einer erfindungsgemäßen Systemanordnung.

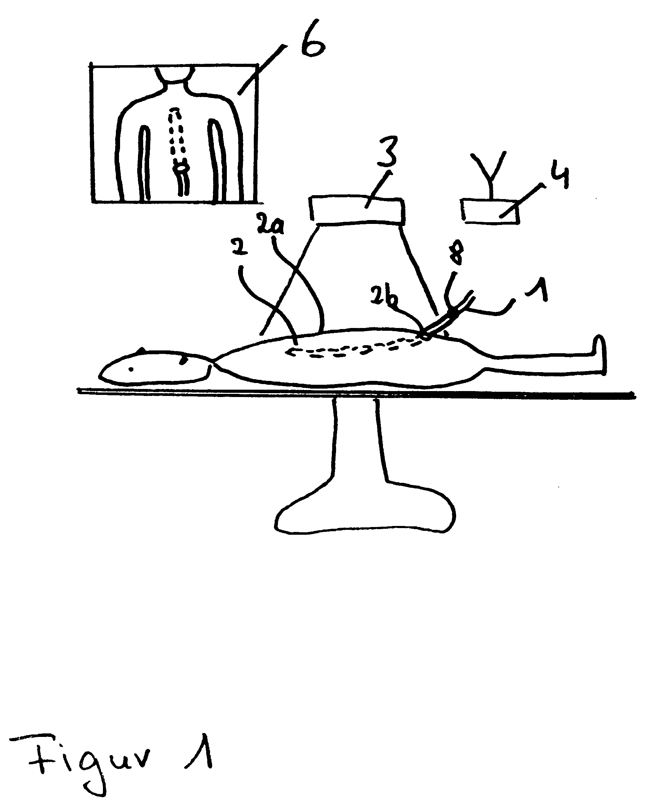

Fig. 1 zeigt eine Ausführung des erfindungsgemäßen Systems anhand einer schematischen Darstellung einer Eingriffssituation in einem Körper 2 eines Patienten. Gemäß diesem Ausführungsbeispiel ist ein Endoskop 1 in einen menschlichen Körper 2 im Bereich des Bauchnabels eines Patienten eingeführt. Das Endoskop erstreckt sich im Innern des Körpers 2 vom Bauchnabel bis in den Bereich des Herzens, wo das distale Ende des Endoskops 1 zum Liegen kommt. Gerade während des Endoskopiervorganges d.h. zu Beginn des Einführens des Endoskops in eine Körperöffnung (Eintrittskanal) und Vorschieben des Endoskops zum Ort des Krankheitsgeschehens wird mittels einer extrakorporalen Kameravorrichtung 3 der Patient bzw. die Oberfläche des Patienten erfasst. Dabei ist die Kameravorrichtung 3 direkt über dem Patienten angeordnet und mit einem entsprechenden Optik versehen, die es ermöglicht, während der Dauer des Eingriffs den für den Operateur wichtigen Teil der Oberfläche es Patienten verlässlich zu erfassen.

Eine solche Anordnung und Ausgestaltung eines Kameravorrichtung hat den Vorteil, dass sie typisch nur einmal zu Beginn eines Eingriffs auf den jeweiligen Körper und dessen individuelle Oberfläche justiert werden muss, so dass stets scharfe, helle und aussagekräftige Bilddaten erzeugt werden, die eine weitere Auswertung durch die Auswerteeinheit 5 ermöglicht..

Das erfindungsgemäße System weist weiterhin ein Lagemeßsytem 4 zur Erfassung der Position und Orientierung des mit Sensoreinheiten 14 versehenen Endoskops 1 auf. Das Lagemeßsystem 4 zeigt Sensoreinheiten 14, die auf oder in das Endoskop 1 integriert angeordnet sind und die mit extrakorporalen Einheiten zusammenwirken und daraus Informationen zur Lage der Sensoreinheiten 14 und folglich des Endoskops 1 ableiten. Die Einheiten des Lagemeßsystems 4 verwenden elektromagnetische Strahlung zur Bestimmung der Lage.

Die Frequenz dieses elektromagnetischen Wechselfeldes ist dabei so bemessen, dass es den menschlichen Körper weitgehend störungsfrei durchdringen kann. Zum anderen sind die Sensoreinheiten 14 so dimensioniert, dass das elektromagnetische Feld von diesen Sensoreinheiten 14 mit gutem Wirkungsgrad erfasst werden kann, so dass massive Störungen der Signale nicht zu befürchten sind und damit eine verlässliche Bestimmung der Lage gewährleistet ist. Die Signale des Lagemeßsystems 4 werde der Auswerteeinrichtung zur weiteren Verarbeitung zugeführt.

Eine in Figur 1 nicht näher dargestellte Auswerteeinrichtung 5 verknüpft rechnerisch die Signale des Lagemeßsystems 4 mit den Bilddaten 2.3 der Körperoberfläche 2a des Patienten der Kameravorrichtung 3. Dabei werden aus den Bilddaten Informationen zur Größe und zur Lage bzw. Orientierung des Körpers 2 des Patienten bestimmt. Die Signale des Lagemeßsystems 4 werden dazu verwendet 3D-Lagedaten zu generieren und diese in ein virtuelles Bild des Endoskops 1 und hier gerade des in den Körper 2 des Patienten eingebrachten Abschnitt des Endoskops 1 zu erzeugen. Dieses virtuelle Bild wird dabei in der Orientierung und Größe so angepasst, dass es mit den Bilddaten zu einem gemeinsamen Bild verbunden wird. Es entsteht ein Augmented-Reality-Bild aus einer Ansicht des Körpers 2 des Patienten mit lage-, größen- sowie orientierungsrichtigem virtuellen Abbild des Verlaufs des Endoskops 1 im Körper 2. Das virtuelle Abbild des Endoskops 1 ist dabei zur Unterscheidung zum reellen Abbild gestrichelt dargestellt. Der durch die Kameravorrichtung 3 erfasste proximale Teil des Endoskops 1 ist nicht gestrichelt dargestellt und weist einen Referenzmarker 8 zur Referenzierung auf. Im Bereich der Körperöffnung 2b, durch die das Endoskop 1 eingeführt wird, stoßen die beiden unterschiedlichen Abbilder des Endoskops 1 aneinander.

Unter Verwendung von Bezugspunkten, die von dem Lagemeßsystem 4 und von der Kameravorrichtung 3 gemeinsam und zugleich erfasst werden, gelingt es sehr rechnerleistungssparend und damit schnell die oben genante richtige Verknüpfung der Bilddaten mit dem virtuellen Bild des Endoskops 1 in der Auswerteeinheit 5 zu schaffen. Diese Bezugspunkte sind quasi Fixpunkte bei der Verknüpfung der Daten. Um diese herum orientieren sich die anderen Daten mit korrekter Lage, Orientierung und Größe. Besonders vorteilhaft werden 2 solchen Bezugspunkte verwendet.

Das Ergebnis der Verknüpfung wird einer Ausgabeeinheit, beispielsweise einem Monitor 6, zugeführt und dort zur Darstellung gebracht. Dieser befindet sich in Sichthöhe des Operateurs.

Die auf dem Monitor 6 dargestellten Bilder ermöglichen es dem Operateur sich sehr schnell zu orientieren. Dies ist gerade bei schwierigen, stressigen Operationen sehr wichtig. Auch bei weniger erfahrenen Operateuren ist durch die erfindungsgemäß verbesserte Orientierungsmöglichkeit ein verbessertes Operationsresultat ermöglicht. Durch die erfindungsgemäße Verknüpfung des auf Basis der 3D-Lagedaten erzeugten virtuellen Bildes des Instrumentes mit den durch die extrakoporale Kameravorrichtung 3 erfassten Bilddaten der Körpers 2 des Patienten gelingt es, eine schnelle und verlässliche Erfassung der Lage des Instrumenten im Körper intuitiv zu erreichen und dadurch Fehlhandlungen eines Operateurs aufgrund mangelnder Orientierung im Körper bei der Operation zu vermeiden. Fehlbehandlungen können hierdurch verringert werden.

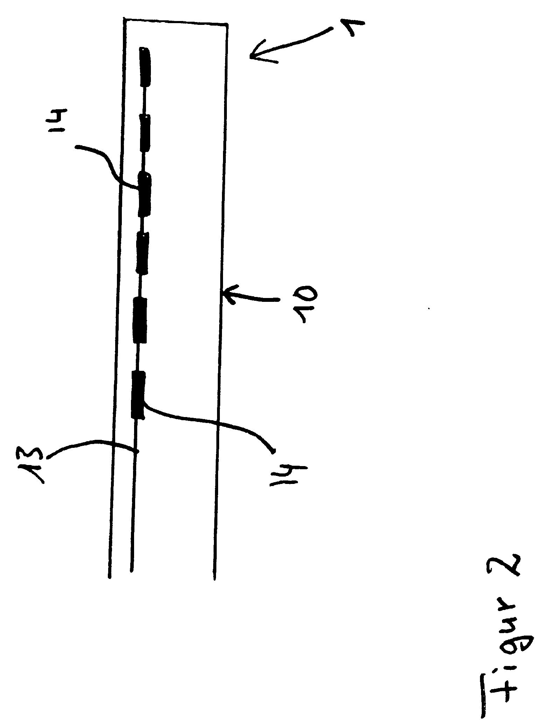

Fig. 2 zeigt ein medizinisches Instrument, ein Endoskop 1, mit mehreren, in Reihe und in den Schaft 10 des Endoskops integriert angeordnete, zylinderförmige Sensoreinheiten 14. Durch die Integration ist eine Sterilisierung des Instrumentes ermöglicht, was die vertraute Handhabung des Operateurs besonders befördert und dadurch Fehlbenutzungen verringert. Diese Sensoreinheiten 14 sind als kleine elektromagnetische Empfangsspulen ausgebildet. Über eine Datenleitung 13 sind die Sensoreinheiten 14 miteinender verbunden und können über diese mit Energie versorgt werden. Die Sensoreinheiten 14 erfassen ein äußeres elektro-magnetisches Feld, das von extrakorporalen Sendeeinheiten, die als Sendespulen realisiert sind, gebildet wird. In Kenntnis des Verlaufs des elektromagnetischen Feldes lassen sich, die Positionen der Sensoreinheiten 14 im Raum bestimmen. Dies erfolgt unter Verwendung von Feldstärkeinformationen und/oder Laufzeitinformationen. Die Sensoreinheiten 14 sind dabei gleichmäßig über einen Abschnitt des Schaftes 10 des Endoskops 1 angeordnet, so dass dessen räumlicher Verlauf gut bestimmt werden kann. Der Abschnitt ist im Bereich des distalen Endes des Schaftes 10 angeordnet, was die besonders relevante Lage des Arbeitsbereiches des Endoskops 1 besonderes verlässlich erfassen lässt und dadurch eine Orientierung des Operateurs bei Bedarf verbessert.

Statt der gezeigten zylinderförmigen Sensoreinheiten 14 können, auch kugel- oder sattelförmig gestaltet Sensoreinheiten 14 gewählt werden und aus flexiblen Materialien gefertigt sein, die es ermöglichen, dass eine Sensoreinheit 14 sich der Gestalt des Endoskops 1 anpasst und dabei insbesondere sich ein seiner Form der Krümmung des Instruments 1 im Körper 2 anpasst. Zur Bestimmung der Lage einer Sensoreinheit 14 muss eine Sensoreinheit nicht notwendigerweise Signale nur empfangen können, sondern kann auch dergestalt ausgebildet sein, dass sie selbst Signale zur Lagebestimmung senden und/oder zusätzlich empfangen kann.

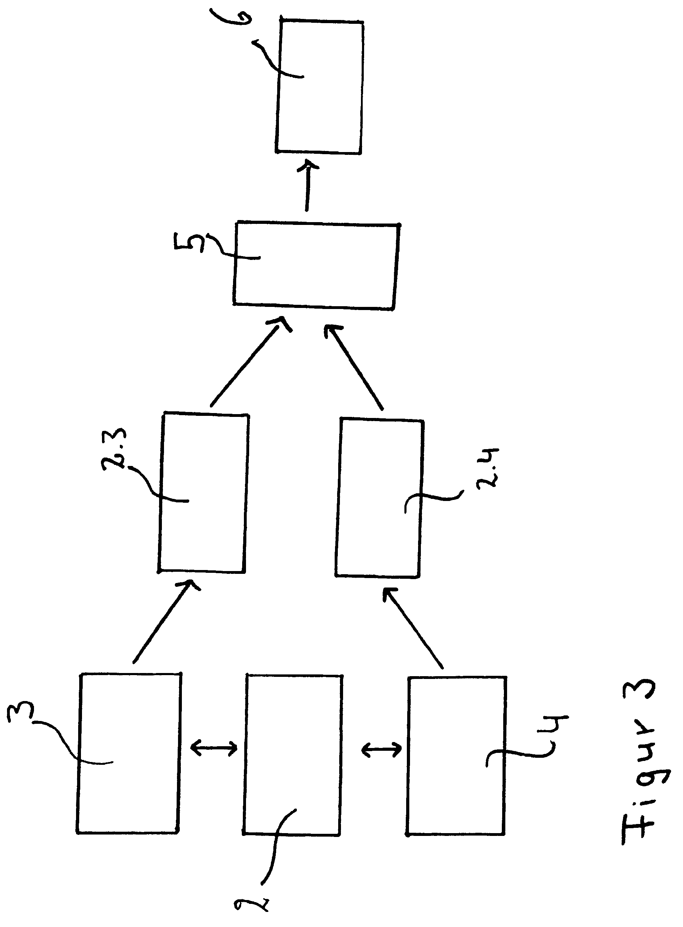

Fig. 3 zeigt in Form eines Blockschaltbildes ein Beispiel für den Aufbau des vorgeschlagenen Systems. Das System weist im dargestellten Beispiel folgende Einheiten bzw. Komponenten auf: eine externe Kameravorrichtung 3, ein Lagemeßsystem 4, eine Auswerteeinheit 5 und eine Ausgabeeinheit 6.

Die Auswerteeinheit 5 ist derart ausgebildet, dass ihr über zwei Schnittstellen Bilddaten 2.3 und 3D-Lagedaten 2.4 zugeführt werden können. Die Bilddaten 2.3 stammen von einer Kameravorrichtung 3, die wenigstens einen Teil der Oberfläche des Körpers erfasst und daraus Bilddaten 2.3 der erfassten Oberfläche generiert. Die 3D-Lagedaten werden von einem Lagemeßsystem 4 bereitgestellt. Das Lagemeßsystem 4 ermittelt eigenständig die Lage des mit mehreren Sensoreinheiten 14 versehenen Instruments 1 und generiert entsprechende 3D-Lagedaten 2.4..

Die Auswerteeinheit 5 ist erfindungsgemäß derart ausgebildet, dass es aus der Mehrzahl durch das Lagemeßsystem 4 erfassten 3D-Lagedaten 2.4 der Sensoreinheiten 14 zumindest ausschnittsweise ein virtuelles 3D-Modell des Endoskops 1 erstellen kann aus dem ein virtuelles 2D-Bild des interventionellen Instruments abgeleitet wird. Des Weiteren werden die durch die Kameravorrichtung 3 generierten Bilddaten mit den individuellen 3D-Lagedaten zur Erstellung eines gemeinsamen Augmented-Reality-Bildes miteinander verknüpft um eine geeignete Bilddarstellung zu generieren.

Die Auswerteeinheit 5 ist mit einer Ausgabeeinheit 6, beispielsweise einem Monitor 6, verbunden, an dem das generierte Augmented-Reality-Bild dargestellt werden kann. Mit diesem System lassen sich in Echtzeit Kamerabilddaten 2.3 mit perspektivischen, visualisierten 3D-Lagedaten 2.4 optimal miteinander verknüpfen und optisch auf einer Ausgabeeinheit 6 darstellen. Das dargestellte System besteht dabei aus standardisierten Komponenten, die sich einfach und verlässlich zu einem gemeinsamen System verbinden lassen. Der Operateur erhält somit ein System zur Orientierungsunterstützung zur Verfügung gestellt, mit dem Eingriffe potentiell schneller und für den Patienten sicherer durchgeführt werden können.

## Patentansprüche

1. System zur Orientierungsunterstützung und Darstellung eines Instruments im Inneren eines Untersuchungsobjektes insbesondere eines Endoskops (1) im menschlichen oder tierischen Körper (2) aufweisend:
ein Lagemeßsystem (4), zur Ermittlung der 3D-Lagedaten des Instruments (1) und eine Ausgabeeinheit (6) zur Darstellung der durch eine Auswerteeinheit (5) generierten Bilder, eine Datenerfassungseinheit mit einer außerhalb des Untersuchungsobjektes angeordnete Kameravorrichtung (3) aufweist, die wenigstens einen Teil der Oberfläche (2a) des Untersuchungsobjekts erfasst und Bilddaten der erfassten Oberfläche (2a) generiert,
eine Auswerteeinheit (5), die 3D Lagedaten mit den Bilddaten so verknüpft, dass ein den Bilddaten lage- und größenentsprechendes Bild des Instruments (1) generiert wird
und eine Ausgabeeinheit (6) die einen Monitor (6) aufweist, mittels dem durch die Auswerteeinheit (5) generierten Bilder des Instruments (1) und Bilder der Kameravorrichtung (3) gemeinsam auf einem Monitor (6) lage-und größenentsprechend dargestellt werden.
**dadurch gekennzeichnet,**
**dass** die Ausgabeeinheit (6) eine Proiektionseinheit aufweist, mittels der die durch die Auswerteeinheit (5) generierten lage- und größenentsprechenden Bilder des Instruments (1) auf eine Projektionsfläche projiziert werden.

2. System nach Anspruch 1, **dadurch gekennzeichnet, dass** die Projektionsfläche durch die Oberfläche (2a) des Untersuchungsobjekts gebildet wird.

3. System nach einem der vorstehenden Ansprüche 1 bis 2, **dadurch gekennzeichnet, dass** eine Einheit zur Erfassung des Oberflächenverlaufs des Untersuchungsobjekts vorgesehen ist und die Auswerteeinheit (5) dazu ausgebildet ist, die Bilddaten an den Oberflächenverlauf der Projektionsfläche anzupassen.

4. System nach einem der vorstehenden Ansprüche 1 bis 3, **dadurch gekennzeichnet, dass** das Lagemeßsystem (4) wenigstens eine insbesondere mehr als 3 Sensoreinheiten (14) aufweist, die in oder an dem Instrument (1) angeordnet sind.

5. System nach Anspruch 4, **dadurch gekennzeichnet, dass** die Sensoreinheiten (14) zum Erzeugen und/oder Detektieren eines elektromagnetischen Feldes ausgestaltet sind und die Positionen der Sensoreinheiten (14) anhand des vom Lagemeßsystem (4) erzeugten oder detektierten elektromagnetischen Feldes bestimmt wird.

6. System nach einem der vorstehenden Ansprüche 4 bis 5, **dadurch gekennzeichnet, dass** mehrere Sensoreinheiten (14) gleichmäßig über einen Abschnitt des Instrumentes (1) verteilt auf oder in dem Instrument (1) angeordnet sind.

7. System nach einem der vorstehenden Ansprüche 4 - 7, **dadurch gekennzeichnet, dass** mehrere Sensoreinheiten (14) über einen Abschnitt des Instrumentes (1) ungleichmäßig, insbesondere im distalen Endbereich des Instrumentes (1) mit höherer Dichte angeordnet sind.

8. System nach einem der vorstehenden Ansprüche 1 bis 7, **dadurch gekennzeichnet, dass** das Lagemeßsystem (4) wenigstens einen vom Instrument (1) räumlich getrennt insbesondere außerhalb des Untersuchungsobjekts angeordneten Referenzmarker aufweist, sodass er insbesondere von der Datenerfassungseinheit (3) und dem Lagemeßsystem (4) und/oder der Einheit zur Erfassung des Oberflächenverlaufs zur Referenzierung erfasst werden kann.

9. System nach Anspruch 8, **dadurch gekennzeichnet, dass** Referenzmarker an der Oberfläche (2a) des Untersuchungsobjekts angeordnet sind.

10. System nach Anspruch 8, **dadurch gekennzeichnet, dass** Referenzmarker extrakorporal vom Untersuchungsobjekts beabstandet insbesondere an einer Wand eines Untersuchungsraums angeordnet sind

11. System nach einem der vorstehenden Ansprüche 1 bis 10, **dadurch gekennzeichnet, dass** das Instrument (1) ein Katheter, ein starres oder ein flexibles Endoskop, ein Instrument zur endoskopischen Chirurgie, eine endoskopische Kapsel oder ein Implantat darstellt.

12. System nach einem der vorstehenden Ansprüche 1 bis 11, **dadurch gekennzeichnet, dass** die Datenerfassungseinheit (3) und das Lagemeßsystem (4) synchron ihre Daten erfassen und insbesondere die Auswertung und/oder Ausgabe diskontinuierlich erfolgt.

13. System nach einem der vorstehenden Ansprüche 1 bis 11, **dadurch gekennzeichnet, dass** die Datenerfassungseinheit (3) und das Lagemeßsystem (4) kontinuierlich ihre Daten erfassen und die Auswertung und/oder Ausgabe der Daten zeitkorrekt insbesondere in Echtzeit erfolgt.

14. System nach einem der vorstehenden Ansprüche 1 bis 13, **dadurch gekennzeichnet**, das die Auswerteeinheit (5) so ausgebildet ist, dass gemeinsam im Bild der Kameravorrichtung (3) und in den 3D-Lagedaten des Instrumentes (1) vorhandene extrakoporale Positionen des Instrumentes (1) als Bezugspunkte für die Generierung des lage- und größenentsprechenden Bildes des Instruments (1) verwendet wird.

## Claims

1. System for orientation support and imaging an instrument in the interior of an examined object, in particular an endoscope (1) in the human or animal body (2), comprising:
a position measurement system (4) for establishing the 3D position data of the instrument (1) and an output unit (6) for displaying the images generated by an evaluation unit (5), a data detection unit which has a camera device (3) arranged outside of the examined object, which detects at least some of the surface (2a) of the examined object and generates image data of the detected surface (2a),
an evaluation unit (5), which links the 3D position data to the image data in such a way that an image of the instrument (1) corresponding to the image data in terms of position and size is generated,
and an output unit (6) which has a monitor (6), by means of which images of the instrument (1) generated by the evaluation unit (5) and images of the camera device (3) are displayed together on a monitor (6) in a manner corresponding in terms of position and size,
**characterized**
**in that** the output unit (6) has a projection unit, by means of which the images of the instrument (1) corresponding in terms of position and size, generated by the evaluation unit (5) are projected onto a projection area.

2. System according to Claim 1, **characterized in that** the projection area is formed by the surface (2a) of the examined object.

3. System according to either of the preceding Claims 1 and 2, **characterized in that** a unit for detecting the surface profile of the examined object is provided and the evaluation unit (5) is embodied to adapt the image data to the surface profile of the projection area.

4. System according to one of the preceding Claims 1 to 3, **characterized in that** the position measurement system (4) has at least one sensor unit, in particular more than 3 sensor units (14), which is/are arranged in or on the instrument (1).

5. System according to Claim 4, **characterized in that** the sensor units (14) are embodied to generate and/or detect an electromagnetic field and the positions of the sensor units (14) are determined on the basis of the electromagnetic field generated or detected by the position measurement system (4).

6. System according to either of the preceding Claims 4 and 5, **characterized in that** a plurality of sensor units (14) are arranged on or in the instrument (1) in a manner distributed uniformly over a portion of the instrument (1).

7. System according to one of the preceding Claims 4 to 7, **characterized in that** a plurality of sensor units (14) are arranged over a portion of the instrument (1) in a non-uniform manner, in particular with a higher density in the distal end region of the instrument (1).

8. System according to one of the preceding Claims 1 to 7, **characterized in that** the position measurement system (4) has at least one reference marker which is spatially separated from the instrument (1), in particular arranged outside of the examined object, such that it can be detected for referencing purposes, in particular by the data detection unit (3) and the position measurement system (4) and/or the unit for detecting the surface profile.

9. System according to Claim 8, **characterized in that** reference markers are arranged at the surface (2a) of the examined object.

10. System according to Claim 8, **characterized in that** reference markers are arranged in an extracorporeal manner at a distance from the examined object, in particular at a wall of an examination room.

11. System according to one of the preceding Claims 1 to 10, **characterized in that** the instrument (1) represents a catheter, a rigid or flexible endoscope, an instrument for endoscopic surgery, an endoscopic capsule or an implant.

12. System according to one of the preceding Claims 1 to 11, **characterized in that** the data detection unit (3) and the position measurement system (4) detect their data synchronously and, in particular, the evaluation and/or output takes place in a discontinuous manner.

13. System according to one of the preceding Claims 1 to 11, **characterized in that** the data detection unit (3) and the position measurement system (4) detect their data continuously and the evaluation and/or output of the data takes place at correct times, in particular in real time.

14. System according to one of the preceding Claims 1 to 13, **characterized in that** the evaluation unit (5) is embodied in such a way that extracorporeal positions of the instrument (1), present together in the image of the camera device (3) and in the 3D position data of the instrument (1), are used as reference points for generating the image of the instrument (1) corresponding in terms of position and size.

## Revendications

1. Système d'aide à l'orientation et à la représentation d'un instrument à l'intérieur d'un objet examiné, notamment d'un endoscope (1) dans un organisme humain ou animal (2), comprenant :
un système de mesure de position (4) destiné à déterminer les données de position 3D de l'instrument (1) et une unité de sortie (6) destinée à représenter les images générées par une unité d'évaluation (5), une unité de détection de données avec comporte un dispositif à caméra (3) disposé à l'extérieur de l'objet examiné, lequel dispositif à caméra détecte au moins une partie de la surface (2a) de l'objet examiné et génère des données d'image de la surface (2a) détectée,
une unité d'évaluation (5) qui combine les données de position 3D aux données d'image de manière générer une image de l'instrument (1) de manière correspondante en position et en taille aux données d'image,
et une unité de sortie (6) qui comporte un écran (6) au moyen duquel des images de l'instrument (1) générées par l'unité d'évaluation (5) et des images du dispositif à caméra (3) sont représentées en commun de manière correspondante en position et en taille sur un écran (6),
**caractérisé en ce que** l'unité de sortie (6) comporte une unité de projection au moyen de laquelle les images de l'instrument (1) qui correspondent en position et en taille et qui sont générées par l'unité d'évaluation (5) sont projetées sur une surface de projection.

2. Système selon la revendication 1, **caractérisé en ce que** la surface de projection est formée par la surface (2a) de l'objet examiné.

3. Système selon l'une quelconque des revendications 1 et 2, **caractérisé en ce qu'**il est prévu une unité destinée à détecter la courbe de surface de l'objet examiné et **en ce que** l'unité d'évaluation (5) est conçue pour adapter les données d'image à la courbe de surface de la surface de projection.

4. Système selon l'une quelconque des revendications 1 à 3, **caractérisé en ce que** le système de mesure de position (4) comporte au moins une unité de détection et plus particulièrement plus de 3 unités de détection (14) qui sont disposées dans ou sur l'instrument (1).

5. Système selon la revendication 4, **caractérisé en ce que** les unités de détection (14) sont conçues pour générer et/ou détecter un champ électromagnétique et **en ce que** les positions des unités de détection (14) sont déterminées sur la base du champ électromagnétique généré ou détecté par le système de mesure de positon (4).

6. Système selon l'une quelconque des revendications 4 à 5 précédentes, **caractérisé en ce que** plusieurs unités de détection (14) sont réparties régulièrement sur une section de l'instrument (1) ou sont disposées dans l'instrument (1).

7. Système selon l'une quelconque des revendications 4-7, **caractérisé en ce que** plusieurs unités de détection (14) sont disposées irrégulièrement avec une densité plus élevée sur une section de l'instrument (1), notamment dans la région d'extrémité distale de l'instrument (1).

8. Système selon l'une quelconque des revendications 1 à 7, **caractérisé en ce que** le système de mesure de position (4) comporte au moins un repère de référence disposé de manière spatialement séparée de l'instrument (1), notamment à l'extérieur de l'objet examiné, de manière à ce qu'il puisse être détecté, notamment par l'unité de détection de données (3) et le système de mesure de position (4) et/ou par l'unité destinée à détecter la courbe de surface à des fins de détermination de référence.

9. Système selon la revendication 8, **caractérisé en ce que** des repères de référence sont disposés sur la surface (2a) de l'objet examiné.

10. Système selon la revendication 8, **caractérisé en ce que** des repères de référence sont disposés de façon extracorporelle en étant espacés de l'objet examiné, notamment sur une paroi d'un espace examiné.

11. Système selon l'une quelconque des revendications 1 à 10, **caractérisé en ce que** l'instrument (1) représente un cathéter, un endoscope rigide ou flexible, un instrument de chirurgie endoscopique, une capsule endoscopique ou un implant.

12. Système selon l'une quelconque des revendications 1 à 11 précédentes, **caractérisé en ce que** l'unité de détection de données (3) et le système de mesure de position (4) détectent leur données de manière synchrone, notamment, **en ce que** l'évaluation et/ou la sortie s'effectuent de manière discontinue.

13. Système selon l'une quelconque des revendications 1 à 11 précédentes, **caractérisé en ce que** l'unité de détection de données (3) et le système de mesure de position (4) détectent leurs données de manière continue et **en ce que** l'évaluation et/ou la sortie s'effectuent de manière corrigée dans le temps, notamment en temps réel.

14. Système selon l'une quelconque des revendications 1 à 13, **caractérisé en ce que** l'unité d'évaluation (5) est conçue de manière à utiliser en commun dans l'image du dispositif à caméra (3) et dans les données de position 3D de l'instrument (1) des positions extracorporelles présentes de l'instrument (1) en tant que points de référence pour générer l'image de l'instrument (1) de manière correspondante en position et en taille.
